(19) Europäisches Patentamt
European Patent Office
Office européen des brevets

(11) Veröffentlichungsnummer: **0 091 556**
**B1**

(12) **EUROPÄISCHE PATENTSCHRIFT**

(45) Veröffentlichungstag der Patentschrift: **26.09.90**

(51) Int. Cl.⁵: **G 01 N 24/08, A 61 B 5/05**

(21) Anmeldenummer: **83102170.4**

(22) Anmeldetag: **05.03.83**

(54) Verfahren zum Messen der magnetischen Kernresonanz für die NMR-Tomographie.

(30) Priorität: **18.03.82 DE 3209810**

(43) Veröffentlichungstag der Anmeldung:
**19.10.83 Patentblatt 83/42**

(45) Bekanntmachung des Hinweises auf die Patenterteilung:
**26.09.90 Patentblatt 90/39**

(84) Benannte Vertragsstaaten:
**AT BE CH DE FR GB IT LI LU NL SE**

(56) Entgegenhaltungen:
EP-A-0 086 306      GB-A-2 113 399
GB-A-2 026 172
JOURNAL OF PHYSICS E: SCIENTIFIC INSTRUMENTS, Band 15, Nr. 1, Januar 1982, Seiten 74-79, The Institute of Physics, Dorking, GB; G. JOHNSON et al.: "Instrumentation for NMR spin-warp imaging"
JOURNAL OF PHYSICS E: SCIENTIFIC INSTRUMENTS, Band 13, September 1980, Seiten 947-955, The Institute of Physics, Dorking, GB; J.M.S. HUTCHISON et al.: "A whole-body NMR imaging machine"

(73) Patentinhaber: **Bruker Medizintechnik GmbH**
**Silberstreifen**
**D-7512 Rheinstetten-Forchheim (DE)**

(72) Erfinder: **Post, Hans, Dr.**
**Griethweg 23**
**D-6905 Schriesheim (DE)**
Erfinder: **Ratzel, Dieter**
**Buchenweg 31**
**D-7512 Rheinstetten (DE)**
Erfinder: **Brunner, Peter, Dr.**
**Schumacherstrasse 13**
**D-7505 Ettlingen (DE)**
Erfinder: **Knüttel, Bertold**
**Baumgartenstrasse 5**
**D-7512 Rheinstetten-Moersch (DE)**

(74) Vertreter: **Patentanwälte Kohler - Schwindling - Späth**
**Hohentwielstrasse 41**
**D-7000 Stuttgart 1 (DE)**

(56) References cited:
**PHYSICS IN MEDICINE AND BIOLOGY, Band 25, 12. Mai 1980, Seiten 751-756, London, GB; W.A. EDELSTEIN et al.: "Spin warp NMR imaging and applications to human whole-body imaging"**

## Beschreibung

Die Erfindung betrifft ein Verfahren zum Messen der magnetischen Kernresonanz in ausgewählten Bereichen eines Körpers zum Zweck der Bilddarstellung von Körperquerschnitten (NMR-Tomographie), bei dem die im Bereich einer Körperebene befindlichen Kernspins einer ausgewählten Art einem statischen Magnetfeld und einem überlagerten, sich in der ausgewählten Querschnittsebene ändernden Gradientenfeld ausgesetzt und selektiv angeregt werden und dann die von den angeregten Kernspins gelieferten Induktionssignale rechnerisch zu Bildsignalen verarbeitet werden.

Die von der NMR-Tomographie gelieferten Querschnittsbilder geben im wesentlichen die Dichteverteilung der Kernspins des ausgewählten Art in der Querschnittsebene wieder. Die Signalamplitude, welche die Helligkeit der einzelnen Bildpunkte bestimmt, ist aber nicht nur von der Dichte der angeregten Kernspins abhängig, sondern auch von deren Anregungszustand. Weiterhin wird die Bildqualität in nicht unerheblichem Maße durch unvermeidliche Rauschsignale beeinträchtigt. Daher ist der Informationsgehalt der mittels der NMR-Tomographie erhaltenen Bilder von Körperquerschnitten manchen Unsicherheiten Unterworfen und es haben diese Bilder nicht die wünschenwerte Qualität.

Demgemäß liegt der Erfindung die Aufgabe zugrunde, die bekannten Verfahren zur NMR-Tomographie su zu verbessern, daß der Informationsgehalt vergrößert und die Bildqualität verbessert wird.

Diese Aufgabe wird nach dem Verfahren gemäß Anspruch 1.

Bei Anwendung des erfindungsgemäßen Verfahrens wird bei einer Messung anstelle des bisher üblichen einen Bildes eine Anzahl von Bildern erhalten, die sich in ihrer Struktur nicht unerheblich unterscheiden, weil die Relaxationszeiten $T_1$ und $T_2$ der angeregten Kernspins, die sich in unterschiedlichen Strukturen des Körperquerschnittes befinden, unterschiedlich sind, so daß die Signalintensität der einzelnen Strukturen des Körperquerschnittes im Verlauf der Aufnahmezeit Änderungen erfährt. Dabei können Einzelheiten in den zeitlich aufeinanderfolgenden Bildern verschwinden und durch andere ersetzt werden, die in den ersten Bildern nicht erkennbar waren. Der besondere Vorteil des erfindungsgemäßen Verfahrens besteht darin, daß die dadurch gewonnene, zusätzliche Information nicht mit einer nennenswerten Verlängerung der Meßzeit erkauft wird, die insbesondere bei Messungen im medizinischen Bereich nicht tolerierbar wäre und allgemein bei Untersuchungen in vivo zu Lägeänderungen des untersuchten Objektes mit einem entsprechenden Informationsverlust führen könnte. Anders als bei Bilderfolgen, die durch mehrmalige Wiederholung der Messung gewonnen werden könnten, sind die bei dem nach dem erfindungsgemäßen Verfahren gewonnenen Bilder genau deckungsgleich.

Bei den Impulsfolgen, die bei dem erfindungsgemäßen Verfahren verwendet werden, kann es sich um die gleichen Impulsfolgen handeln, wie sie auch bei der normalen NMR-Spektrographie zur Ermittlung der Relaxationszeiten verwendet werden, also insbesondere im Falle von $T_1$ um die DEFT-Folge (siehe E. D. Becker et al in J. Amer. Chem. Soc., Bd. 91 (1969), S. 7784) oder entsprechende Varianten bzw. im Falle von $T_2$ um eine Carr-Purcell-Folge mit oder ohne Gill-Meiboom-Modifikation. Während jedoch bei der klassischen NMR-Spektroskopie die Signale integral aus dem gesamten Probenvolumen stammen und daher unmittelbar vergleichbar sind, ist ein solcher Vergleich der durch eine solche Impulsfolge angeregten aufeinanderfolgenden Interferogramme nicht möglich, weil die einzelnen Interferogramme die Signale aus großen Volumenbereichen repräsentieren, die keine unmittelbar brauchbaren Daten liefern. Vielmehr bedarf es einer umfangsreichen Bearbeitung der freien Induktionssignale, um die Informationen über die einzelnen Bildpunkte zu gewinnen. Daher konnte es allenfalls sinnvoll erschienen, die durch solche Impulsfolgen gewonnenen Induktionssignale in bekannter Weise vor ihrer Weiterverarbeitung aufzuaddieren, um dadurch das Signal-Rausch-Verhältnis zu verbessern. Die hierdurch erzielbare Verbesserung der Bildqualität ist jedoch sehr viel geringer als diejenige, die mit Hilfe des erfindungsgemäßen Verfahrens erzielbar ist. Darüber hinaus gehen dabei die Informationen über die Relaxationszeiten verloren, die bei dem erfindungsgemäßen Verfahren durch die Maßnahme, die jeweils gemäß ihrer zeitlichen Folge einander entsprechenden Induktionssignale zu einem eigenen Satz von Bildsignalen zu verarbeiten, gewonnen werden. Bekannte Verfahren sind in Dokumenten GB—A—2 026 172 und EP—A—0 086 306 (Prioritätsdatum: 21.12.81, Veröffentlichungsdatum: 24.08.83) beschrieben.

Wie bereits erwähnt, besteht ein besonderer Vorteil des erfindungsgemäßen Verfahrens darin, daß alle Bilder, die zeitlich aufeinanderfolgende Zustände wiedergeben, bei dem gleichen Meßvorgang gewonnen werden, so daß sie absolut deckungsgleich sind und daher einen genauen Vergleich ermöglichen. Das bietet dann auch die Möglichkeit, die den einzelnen Bildpunkten zugeordneten Bildsignale miteinander in Beziehung zu setzen und dadurch aus der Variation der den einzelnen Bildpunkten zugeordneten Bildsignale die Relaxationszeiten $T_1$ bzw. $T_2$ für die einzelnen Volumenelemente des Körperquerschnittes zu ermitteln, die den bildpunkten zugeordnet sind. Da das erfindungsgemäße Verfahren in der Lage ist, eine relativ große Anzahl von zeitlich aufeinanderfolgenden Werten für jeden einzelnen Bildpunkt zu liefern, beispielsweise 24 solcher Werte, ist es möglich, daraus die für die Relaxationszeiten $T_1$ bzw. $T_2$ charakteristischen e-Funktionen mit hoher Genauigkeit zu ermitteln. Entsprechend genau ist auch die Ermittlung der den einzelnen Bildpunkten zugeordneten Relaxationszeiten. Da festgestellt wurde, daß die Relaxationszeiten $T_1$

und $T_2$ von angeregten Kernspins im Krebsgewebe erheblich größer sind als diejenige von Kernspins in gleichartigem, gesundem Gewebe, kommt einer genauen Ermittlung der Relaxationszeiten $T_1$ und $T_2$ eine erhebliche Bedeutung zu. Da das erfindungsgemäße Verfahren die Möglichkeit gibt, anhand der gewonnenen Bilder unterschiedliche Relaxationszeiten zu erkennen, dürften sich hier interessante Perspektiven zur Krebsdiagnostik ergeben, da nicht mehr die Notwendigkeit besteht, einzelne Gewebeproben in vitro gesondert zu untersuchen.

Da es das erfindungsgemäße Verfahren ermöglicht, die Relaxationszeit $T_1$ und $T_2$ für die einzelnen Volumenelemente des Körperquerschnittes sehr genau zu ermitteln, besteht auch die Möglichkeit, in weiterer Ausgestaltung der Erfindung einen Satz Bildsignale zu erzeugen, der nur die Dichte von Kernspins mit einer ausgewählten Relaxationszeit $T_1$ bzw. $T_2$ wiedergibt.

Wäre beispielsweise die Spin-Spin-Relaxationszeit $T_2$ eines typischen Krebsgewebes bekannt, so bestünde die Möglichkeit, nach dem erfindungsgemäßen Verfahren Bilder zu erzeugen, die ausschließlich solche Körperstellen wiedergeben, die Krebsgewebe enthalten. So wäre es beispiels denkbar, bei einer operativen Entfernung von Krebsgewebe die Relaxationszeit $T_2$ dieses Gewebes festzustellen und dann den Patienten mit Hilfe des erfindungsgemäßen Verfahrens darauf zu untersuchen, ob in seinem Körper und insbesondere in der Umgebung des Operationsfeldes Gewebe mit der gleichen Relaxationszeit $T_2$ wieder auftritt.

Die Möglichkeit, wegen der genauen Übereinstimmung der einzelnen Punkte des gewonnenen Bildersatzes die den einzelnen Bildpunkten zugeordneten Relaxationszeiten $T_1$ bzw. $T_2$ mit hoher Genauigkeit zu bestimmen, bietet auch einen experimentellen Weg, eine Korrektur sämtlicher Bildpunkte der einzelnen Bilder in der Weise vorzunehmen, daß ihre Intensität genau auf denjenigen Wert gebracht wird, den sie gemäß der e-Funktion haben muß, die sich aus der ermittelten Relaxationszeit ergibt. Auf diese Weise lassen sich Störungen durch Rauschsignal und dergleichen stark reduzieren, so daß ohne Verlängerung der Meßzeit Bilder mit einer Qualität gewonnen werden, die notwendig besser ist, als sie durch die üblichen Methoden zur Signal-Rausch-Verbesserung erreichbar ist.

Das erfindungsgemäße Verfahren hat zur Voraussetzung, daß die mittels der Impulsfolge im Bereich der ausgewählten Körperebene anzuregenden Kernspins noch keinen Einflüssen ausgesetzt waren, die ihre Ausrichtung zum homogenen statischen Magnetfeld gestört haben, so daß sie bei einer aus 90°- und 180°-Impulsen bestehenden Impulsfolge immer wieder eine zum statischen Magnetfeld parallele Ausrichtung erhalten, damit sie eine Vielzahl aufeinanderfolgender Induktionssignale liefern können. Im einfachsten Fall kann das Verfahren zur Untersuchung eines von vornherein scheibenförmigen Körpers verwendet werden, dessen gesamtes Volumen das zu erzeugende Bild liefert und der daher keinen Vorgängen ausgesetzt zu werden braucht, um eine bestimmte Querschnittsebene für die Bildaufnahme zu präparieren. Das erfindungsgemäße Verfahren ist auch zur Untersuchung von organischen und anorganischen Körpern durchaus interessant, die ohne weiteres mechanisch in Scheiben zerteilt werden können, von denen NMR-Bilder erzeugt werden sollen, die Aufschluß über die Struktur der Körper liefern.

Es besteht aber auch durchaus die Möglichkeit, durch die Anwendung spezieller Anregungsschritte einen Körper in solcher Weise zu präparieren, daß nur noch die in der ausgewählten Körperebene enthaltenen Kernspins parallel zum statischen Magnetfeld ausgerichtet sind und daber alleine noch für eine Anregung zur Verfügung stehen, die eine definiertes Signal liefert. Demgemäß wird in weiterer Ausgestaltung der Erfindung die Selektion der Körperebene durch die Anwendung eines zum homogenen Magnetfeld parallelen Gradientenfeldes, das sich in einer zur ausgewählten Ebene senkrechten Richtung ändert, und ausgewählte Pulssignale bewirkt, die sowohl selektiv die in der ausgewählten Ebene enthaltenen Kernspins der ausgewählten Art als auch die Gesamtheit der ausgewählten Kernspins derart anregen, daß im wesentlichen nur die in der ausgewählten Ebene enthaltenen Kernspins in die Richtung des statischen Magnetfeldes zurückgebracht werden. Ein solches Anregungsverfahren ist Gegenstand der älteren Patentanmeldung DE—A—32 09 264.4, auf deren Inhalt hiermit verwiesen wird, so daß an dieser Stelle dieses Verfahren nicht im einzelnen erläutert zu werden braucht.

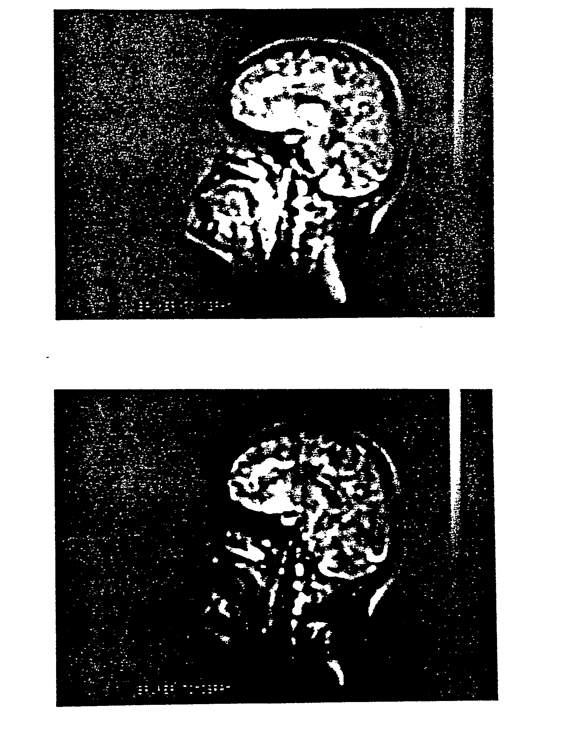

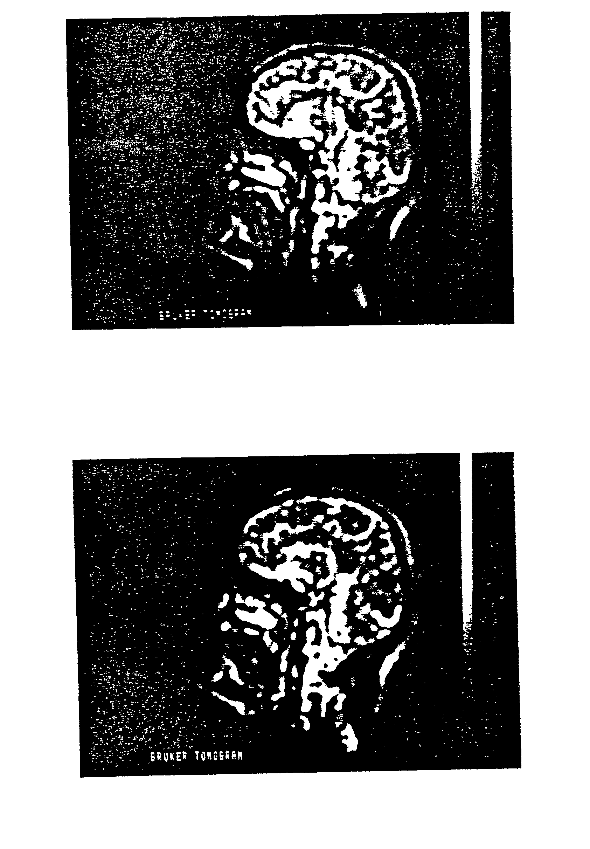

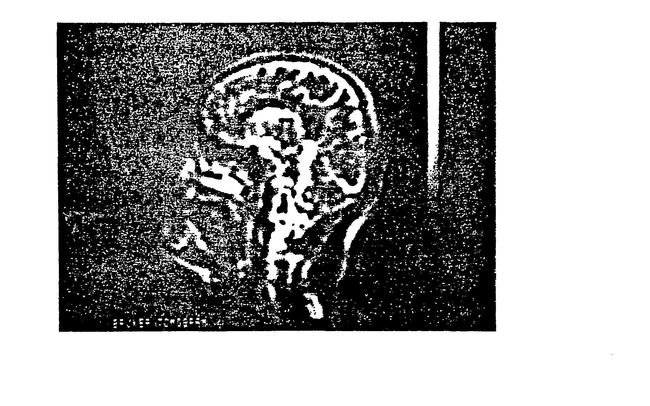

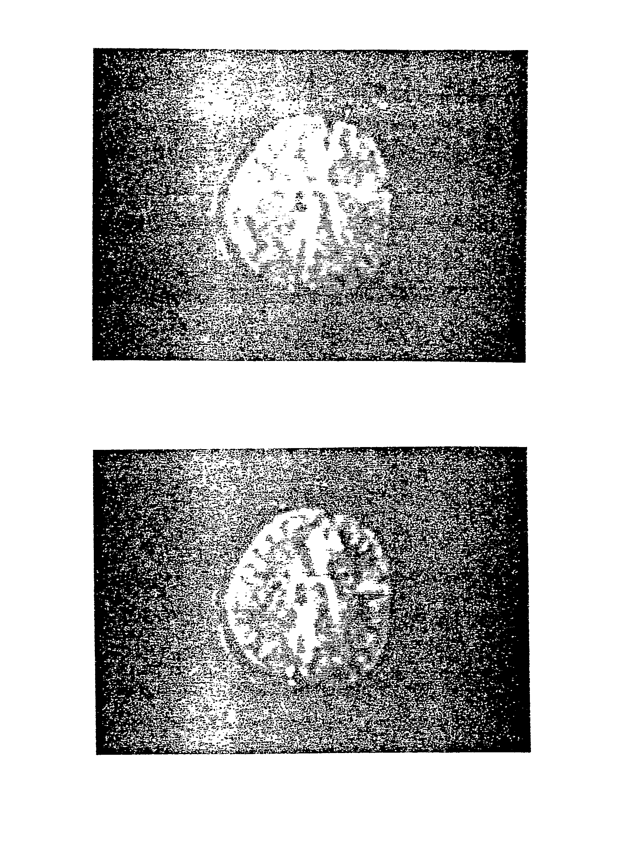

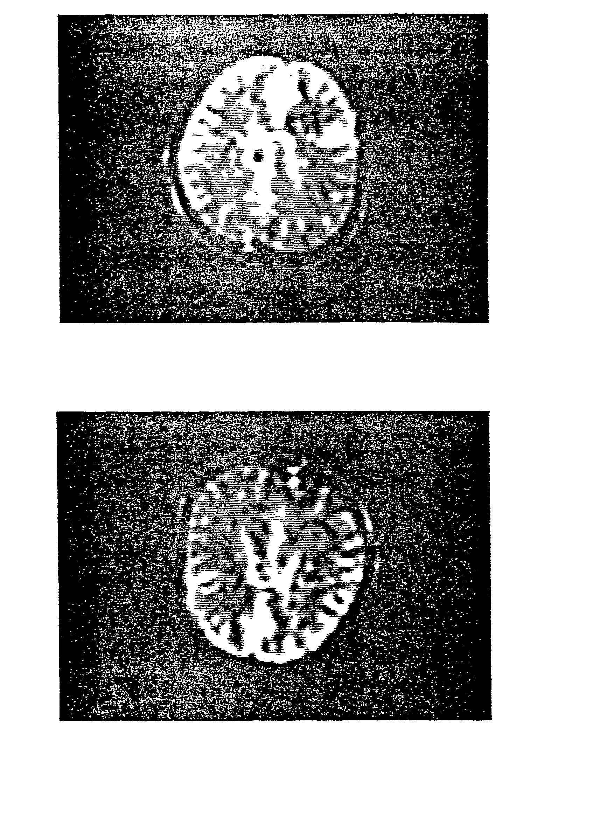

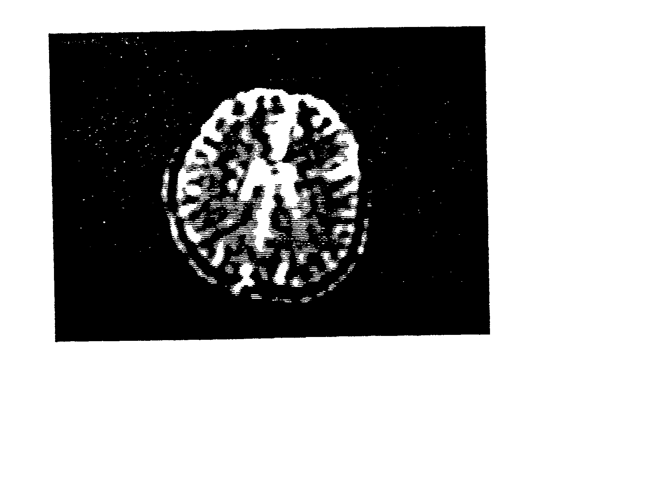

Um die Bedeutung des erfindungsgemäßen Verfahrens zu veranschaulichen, sind zwei Sätze von Fotografien beigefügt, welche die Folge von Querschnittsbildern wiedergeben, die nach dem erfindungsgemäßen Verfahren erhalten worden sind. Es handelt sich um die tomographische Erfassung einer sagittalen sowie einer transversalen Querschnittsebene durch einen menschlichen Kopf. Die Selektion der Querschnittsebenen erfolgte nach dem in der soeben genannten Patentanmeldung beschriebenen Verfahren. Die Anregung der ausgewählten Spins von Protonen erfolgte durch eine Carr-Purcell-Folge in solcher Weise, daß das erste Bild 16 ms nach der ersten Anregung entstand und die anderen Bilder jeweils in einem Abstand von weiteren 32 ms folgen. Die Strukturunterschiede zwischen den aufeinanderfolgenden Bildern sind eklatant. Im ersten Bild deutlich vorhandene Strukturen verschwinden langsam und machen anderen Strukturen Platz. Beispielsweise sei das Auftauchen des kleinen hellen runden Bogens am unteren Abschnitt des Hinterkopfes in den sagittalen Schnittbildern sowie das Verschwinden des darunter liegenden, großen dunklen Bogens erwähnt. Ähnlich erfährt die zentrale Struktur des transversalen Schnittbildes erhebliche Wandlungen. In beiden Bilderserien tritt die Abbildung der Gehirnmasse in zunehmendem Maße gegen die Gehirnkontur zurück.

Es sei darauf hingewiesen, daß die beigefügten Bilder noch keineswegs die Qualität haben, die mit dem erfindungsgemäßen Verfahren erzielbar ist, weil einerseits jedes einzelne Bild aus der Aufsummierung von vier aufeinanderfolgenden Bildern entstanden ist und andererseits die obenbehandelte Korrektur anhand der ermittelten $T_2$-Werte noch nicht erfolgt ist.

**Patentansprüche**

1. Verfahren zum Messen der magnetischen Kernrezonanz in ausgewählten Bereichen eines Körpers zum Zwecke der Bilddarstellung von Körperquerschnitten (NMR-Tomographie), bei dem die im Bereich einer Körperebene befindlichen Kernspins einer ausgewählten Art einem statischen Magnetfeld und einem überlagerten, sich in der ausgewählten Querschnittsebene ändernden Gradientenfeld ausgesetzt und selektiv angeregt werden und dann die von den angeregten Kernspins gelieferten Induktionssignale rechnerisch zu Bildsignalen verarbeitet werden, wobei zur Anregung der Kernspins eine der bekannten Impulsfolgen (z.B. DEFT-Folge oder CP-Folge) verwendet wird, dadurch gekennzeichent, daß darauf die in einem Durchgang gelie-ferte Anzahl zeitlich aufeinanderfolgender Induk-tionssignale gemäße ihrer zeitlichen Folge zu einer der Anzahl der aufeinanderfolgenden Induk-tionssignalen entsprechenden Anzahl von Bildsi-gnalen verarbeitet werden, so daß bei einer einzigen Messung einen Folge von deckungsgleichen Querschnittsbildern entsteht, die sich voneinander durch die gemäß der Relaxationszeiten $T_1$ oder $T_2$ variierende Intensität der angeregten Kernspins unterscheiden.

2. Verfahren nach Anspruch 1, dadurch gekennzeichnet, daß aus der Variation der den einzelnen Bildpunkten zugeordneten Bildsignale die Relaxa-tionszeiten $T_1$ bzw. $T_2$ für die einzelnen Volumene-lementen des Körperquerschnittes zugeordneten Bildpunkte ermittelt werden.

3. Verfahren nach Anspruch 2, dadurch gekennzeichnet, daß ein Satz Bildsignale erzeugt wird, der nur die Dichte von Kernspins mit einer ausge-wählten Relaxationszeit $T_1$ bzw. $T_2$ wiedergibt.

4. Verfahren nach Anspruchs oder 3, dadurch gekennzeichnet, daß die Intensität der einzelnen Punkte der aufeinanderfolgenden Bilder gemäß der den ermittelten Relaxationszeiten $T_1$ bzw. $T_2$ entsprechenden e-Funktionen korrigiert wird.

5. Verfahren nach einem der vorhergehenden Ansprüche, dadurch gekennzeichnet, daß die Selektion der Körperebene durch die Anwendung eines zum homogenen Magnetfeld parallelen Gradientfeldes, das sich in einer zur ausgewählten Ebene senkrechten Richtung ändert, und ausgewählter Impulssignale erfolgt, die sowohl selektiv die if der ausgewählten Ebene enthaltenen Kernspins der ausgewählten Art als auch die Gesamtheit der ausgewählten Kernspins derart anregen, daß im wesentlichen nur die in der ausgewählten Ebene enthaltenen Kernspins in die Richtung des homogenen Magnetfeldes zurückgebracht werden.

6. Verfahren nach Anspruch 1, dadurch gekennzeichnet, daß die zur Anregung dienende Impulsfolge nach einem 90° Impuls 24 180° Impulse umfaßt und daß 24 Induktionssignale in einem Durchgang erzeugt werden, die in ihrer zeitlichen Folge der zeitlichen Lage des Impulses zugeordnet werden.

7. Verfahren nach Anspruch 1, dadurch gekennzeichnet, daß die zur Anregung dienende Impulsfolge eine Carr-Purcell-Folge mit Gill-Meiboom Modifikation ist.

**Revendications**

1. Procédé pour mesurer la résonance magnétique nucléaire dans des domaines choisis d'un corps en vue d'obtenir une imagerie de coupes corporelles (tomographie RMN), dans lequel les spins nucléaires d'un type choisi qui se trouvent dans le domaine d'un plan corporel sont exposés à un champ magnétique statique et à un champ de gradient superposé qui varie dans le plan de coupe choisi, et sont excités sélectivement, puis les signaux d'induction délivrés par les spins nucléaires excités sont convertis par le calcul en des signaux d'image, l'une des suites d'impulsions connues (par exemple la suite DEFT ou la suite CP) étant utilisée pour l'excitation des spins nucléaires, caractérisé en ce que le nombre de signaux d'induction consécutifs dans le temps délivrés au cours d'un passage sont convertis selon leur suite temporelle en un nombre de signaux d'image correspondant au nombre des signaux d'induction consécutifs, de sorte que dans le cas d'une seule mesure il apparaît une suite d'images en coupe coïncidentes qui diffèrent les unes des autres par l'intensité des spins nucléaires excités qui varie selon les temps de relaxation $T_1$ ou $T_2$.

2. Procédé selon la revendication 1, caractérisé en ce que les temps de relaxation $T_1$ et $T_2$ pour les points d'image associés à différents éléments volumiques de la coupe corporelle sont déterminés à partir de la variation des signaux d'image associés aux différents points d'image.

3. Procédé selon la revendication 2, caractérisé en ce que l'on produit une série de signaux d'image qui ne restitue que la densité des spins nucléaires présentant un temps de relaxation $T_1$ ou $T_2$ choisi.

4. Procédé selon la revendication 2 ou 3, caractérisé en ce que l'intensité des différents points des images consécutives est corrigée selon les fonctions e correspondant aux temps de relaxation $T_1$ ou $T_2$ déterminés.

5. Procédé selon l'une des revendications précédentes, caractérisé en ce que la sélection du plan corporel se fait par l'utilisation d'un champ de gradient parallèle au champ magnétique homogène, champ de gradient qui varie dans une direction perpendiculaire au plan choisi, et de signaux d'impulsion choisis qui excitent aussi bien de façon sélective les spins nucléaires du type choisi qui sont contenus dans le plan choisi qu'également la totalité des spins nucléaires choisis, de telle façon que sensiblement seuls les spins nucléaires contenus dans le plan choisi soient ramenés dans la direction du champ magnétique homogène.

6. Procédé selon la revendication 1, caractérisé en ce que la suite d'impulsions servant à l'excitation comprend après une impulsion à 90° 24 impulsions à 180° et en ce que 24 signaux d'induction qui, dans leur suite temporelle, sont associés à la position temporelle de l'impulsion, sont produits dans un passage.

7. Procédé selon la revendication 1, caractérisé en ce que la suite d'impulsions servant à l'excitation est une suite de Carr-Purcell avec une modification de Gill-Meiboom.

## Claims

1. Method for measuring the magnetic nuclear resonance in selected areas of a body for the image representation of body cross-sections (NMR tomography), in which the nuclear spins of a selected type located in the vicinity of a body plane are exposed and selectively excited with a static magnetic field and a superimposed vector field varying in the selected cross-sectional plane and then the induction signals supplied by the excited nuclear spins are mathematically processed to image signals and for exciting the nuclear spins use is made of one of the known pulse sequences (e.g. DEFT sequence or CP sequence), characterized in that the number of time-succeeding induction signals supplied in one pass are processed in accordance with their time sequence to a number of image signals corresponding to the number of successive induction signals, so that in a single measurement a sequence of congruent cross-sectional images is obtained, said images differing from one another through the intensity of the excited nuclear spins varying according to the relaxation times $T_1$ or $T_2$.

2. Method according to claim 1, characterized in that the relaxation times $T_1$ and/or $T_2$ for the image points associated with the individual volume elements of the body cross-section are determined from the variation of the image signals associated with the individual image points.

3. Method according to claim 2, characterized in that a set of image signal is produced, which only reproduces the density of nuclear spins with a selected relaxation time $T_1$ and/or $T_2$.

4. Method according to claims 2 or 3, characterized in that the intensity of the individual points of the successive images is corrected in accordance with the exponential functions corresponding to the determined relaxation times $T_1$ and/or $T_2$.

5. Method according to one of the preceding claims, characterized in that the selection of the body plane takes place by using a vector field parallel to the homogeneous magnetic field and which changes in a direction at right angles to the selected plane, as well as selected pulse signals, which selectively excite the nuclear spins of the selected type contained in the selected plane and also all the selected nuclear spins, in such a way that essentially only the nuclear spins contained in the selected plane are returned in the direction of the homogeneous magnetic field.

6. Method according to claim 1, characterized in that the pulse sequence used for excitation comprises after a 90° pulse 24 180° pulses and that 24 induction signals are produced in one pass, whose time sequence is associated with the time position of the pulse.

7. Method according to claim 1, characterized in that the pulse sequence used for excitation is a Carr-Purcell sequence with a Gill-Meiboom modification.